Europäisches Patentamt

European Patent Office

Office européen des brevets

(19)

(11) Publication number: **0 325 782**
**A2**

## (12) EUROPEAN PATENT APPLICATION

(21) Application number: 88121530.5

(22) Date of filing: 22.12.88

(51) Int. Cl.4: **G01N 33/573** , **G01N 33/577** ,
**C12P 21/00** , **C12N 15/00**

The title of the invention has been amended (Guidelines for Examination in the EPO, A-III, 7.3).

(30) Priority: 23.12.87 JP 323763/87

(43) Date of publication of application:
02.08.89 Bulletin 89/31

(84) Designated Contracting States:
BE DE FR GB IT NL

(71) Applicant: **Tosoh Corporation**
**4560, Oaza Tonda**
**Shinnanyo-shi Yamaguchi-ken(JP)**

(72) Inventor: **Gohzu, Shunichi**
**35-21, Sakuradai Midori-ku**
**Yokohama-shi Kanagawa(JP)**
Inventor: **Kondo, Masahide**
**35-21, Sakuradai Midori-ku**
**Yokohama-shi Kanagawa(JP)**
Inventor: **Inouye, Kuniyo**
**37-17-402, Sagamiono 7-chome**
**Sagamihara-shi Kanagawa(JP)**

(74) Representative: **Vossius & Partner**
**Siebertstrasse 4 P.O. Box 86 07 67**
**D-8000 München 86(DE)**

(54) **Method of measurement of Cu,Zn-superoxide dismutase.**

(57) Cu,Zn-superoxide dismutase contained in a sample at a concentration of from 1 ng/ml to 320 ng/ml is measured by performing in one vessel the antigen-antibody reaction of a monoclonal antibody specifically recognizing Cu,Zn-superoxide dismutase, which is immobilized on a solid carrier, and simultaneously therewith, an antigen-antibody reaction of the same Cu,Zn-superoxide dismutase-recognizing monoclonal antibody, which is labelled with a marker.

EP 0 325 782 A2

EP 0 325 782 A2

## MEASUREMENT OF Cu,Zn-SUPEROXIDE DISMUTASE

The present invention relates to a monoclonal antibody specifically recognizing Cu,Zn-superoxide dismutase (hereinafter referred to as "Cu,Zn-SOD") and a method of measuring Cu,Zn-SOD in a sample by using the monoclonal antibody.

Cu,Zn-SOD is an enzyme having a catalytic action for disproportionation reaction by a superoxide radical. The measurement of this enzyme has recently attracted attention in the clinical field and the micromeasurement of Cu,Zn-SOD in various tissues, for example, liver and lung is desired. Moreover, since Cu,Zn-SOD has been used for medicines, the measurement of the concentration of Cu,Zn-SOD in blood is desired. As the conventional measurement method, there is known a method of Mc'Cord et al. (J. Biol. Chem., 244, pages 6049-6055, 1969). In this method, however, the operation is complicated and the measurement sensitivity is not high. Moreover, the method of Mc'Cord et al. is a method in which the quantity of substances erasing a superoxide radical is measured, and is not a method in which only Cu,Zn-SOD is specifically measured.

The superoxide dismutase (SOD) is divided into a Cu,Zn type, Mn type and Fe type according to the kind of the chelating metal necessary for enzyme activity. Cu,Zn-SOD is contained in the cytoplasm of a eukaryote, and Mn-SOD is contained in a mitochondrion. Furthermore, a superoxide radical itself can also be erased by a living body substance such as cytochrome C, epinephrine or ascorbic acid. Therefore, according to the method of Mc'Cord et al, it is impossible to specifically measure only Cu,Zn-SOD in a sample.

As the means of measuring a trace component in the body fluid, there have been adopted immunoassay methods such as the radioimmunoassay method and the enzyme immunoassay method.

In connection with the method of the immunoassay of Cu,Zn-SOD, several patent references have been published. For example, JP-A-57-102195 discloses a method in which a solution containing enzyme-labelled SOD and a solution containing a specific bonding to SOD are mixed with a sample solution, of which SOD is to be measured, an immunocomplex is removed as the precipitate from the aqueous mixed solution, and the activity of the aqueous solution or precipitate is measured to determine SOD. Furthermore, JP-A-57-102196 and JP-A-58-7560 teach that SOD is measured by the sandwich method using an anti-SOD polyclonal antibody labelled with an enzyme and an anti-SOD polyclonal antibody bonded to an insoluble carrier.

Moreover, JP-A-62-167479 teaches that SOD is measured according to the two-stage sandwich method in which the antigen-antibody reaction is carried out in two stages by using a labelled anti-Cu,Zn-SOD monoclonal antibody and an insoluble carrier to which several anti-Cu,Zn-SOD monoclonal antibodies are bonded.

It is an object of the present invention to provide a method for the immunoassay of Cu,Zn-SOD, in which the measurement can be performed with a good reproducibility at a high sensitivity by using a monoclonal antibody for which the same quality can always be obtained.

In one aspect of the present invention, there is provided a monoclonal antibody specifically recognizing Cu,Zn-superoxide dismutase.

In another aspect of the present invention, there is provided a method of measuring Cu,Zn-superoxide dismutase, which comprises placing in a vessel a sample containing Cu,Zn-superoxide dismutase at a concentration of from 1 ng/ml to 320 ng/ml, and performing in the vessel antigen-antibody reaction of a monoclonal antibody specifically recognizing Cu,Zn-superoxide dismutase, which is immobilized on a solid carrier, and, simultaneously therewith, antigen-antibody reaction of the same Cu,Zn-superoxide dismutase-recognizing monoclonal antibody, which is labelled with a marker.

The monoclonal antibodies of the present invention are preferably gotten from cultured products of a hybridoma obtained by fusing antibody-producing spleen cells of an animal immunized with Cu,Zn-SOD, to myeloma cells to obtain hybridoma producing monoclonal antibody recognizing Cu,Zn-SOD, and culturing the hybridoma and/or cell strains derived therefrom. The hybridoma producing the monoclonal antibody recognizing Cu,Zn-SOD is prepared by the known cell-fusing method (G. Kohler and C. Milstein, Nature, 256, page 495, 1975).

In the present invention, the monoclonal antibody recognizing Cu,Zn-SOD is obtained according to the above-mentioned method. Part of the thus-obtained monoclonal antibodies have a binding constant larger than $10^8 M^{-1}$. By using the monoclonal antibodies having such a large binding constant, the measurement of Cu, Zn-SOD can be effected at a higher sensitivity. Since Cu,Zn-SOD is a dimer comprising equivalent

2

sub-units, the solid phase enzyme immunoassay (enzymelinked immunosorbent assay) can be performed by the sandwich method using the same monoclonal antibodies. A plurality of monoclonal antibodies need not be used and one kind of a monoclonal antibody is sufficient. Moreover, since the antigen-antibody reaction can be performed in one and the same vessel by simultaneously using immobilized and labelled antibodies, the operation is much simplified.

In the present invention, the immobilized antibody is formed by immobilizing a monoclonal antibody specifi cally recognizing Cu,Zn-SOD on a solid carrier, and a known method can be adopted for the immobilization.

For the immobilization of the antibody, there are preferably used solid carriers such as beads and microplates of glass, polystyrene, polyethylene, polyvinyl chloride, latex, agarose, cellulose and methacrylate polymer.

The same monoclonal antibody as used for the preparation of the immobilized monoclonal antibody is used for the preparation of the labelled monoclonal antibody. The method or means for labelling the antibody or the method or means for detecting the labelled antibody is not particularly limited, and known methods or means can be adopted. In the method using an enzyme as the marker (EIA), enzymes such as peroxidase, $\beta$-D-galactosidase and alkaline phosphatase can be used. In the method using a radioactive substance (RIA), $^{125}I$ and $^{3}H$ can be used. In the method using a fluorescent substance (FIA), fluorescein isothiocyanate is ordinarily used. Other markers can be used.

When the marker is an enzyme, a substrate is used for measuring the activity of that enzyme. For example, as the substrate for horseradish peroxidase, there can be mentioned 2,2'-adino-di-[3-ethylbenzthi azoline-6-sulfonic acid] diammonium salt (hereinafter referred to as "ABTS")-$H_2O_2$ , 5-aminosalicylic acid-$H_2O_2$ and o-phenylenediamine-$H_2O_2$. As the substrate for $\beta$-D-galactosidase, there can be mentioned o-nitrophenyl-$\beta$-D-galactopyranoside, and as the substrate for alkaline phosphatase, there can be mentioned p-nitrophenyl phosphate.

For the measurement, known reagents such as dissolving agents, washing agents and reaction stopper agents can be used in addition to the above-mentioned reagents.

As is apparent from the foregoing description, according to the present invention, in the sandwich immunoassay method, by using one kind of a monoclonal antibody to Cu,Zn-SOD, two kinds of antigen-antibody reactions can be performed in one vessel simultaneously, and it is so sufficient if each of the operations of adding the reagents and removing unnecessary substances by washing is conducted only once. In short, the measurement can be accomplished by the one-stage sandwich immunoassay method.

In the method of the present invention, the measurement is conducted in the state where the concentration of Cu,Zn-SOD in the sample is in the range of from 1 ng/ml to 320 ng/ml. The reason is that at a concentration outside this range, the measurement becomes difficult according to the existent detection method.

The method of the present invention is a method specific to Cu,Zn-SOD, and the measurement is not influenced at all even if a substance capable of erasing a superoxide radical, other than Cu,Zn-SOD, is present. Moreover, the monoclonal antibody used in the present invention can be produced in a large quantity with a uniform quality, and the reproducibility of the reaction is very high. The monoclonal antibody used in the present invention can be produced on an industrial scale. Furthermore, only one kind of a monoclonal antibody is necessary and the antigen-antibody reaction can be performed in one vessel by simultaneously using the immobilized antibody and labelled antibody. Accordingly, the operation is much simplified and expedited.

The present invention will now be described in detail with reference to the following examples that by no means limit the scope of the invention.

Example 1

(A) Preparation of Monoclonal Antibody to Human Cu,Zn-SOD

The monoclonal antibody to human Cu,Zn-SOD was prepared by the method of G. Kohler and C. Milstein. An emulsion prepared by mixing PBS (phosphate-buffered saline) having, dissolved therein, Cu,Zn-SOD derived from human erythrocytes with Freund's complete adjuvant was injected intraperitonealy into BALB/c mouse twice at an interval of 4 weeks. After rise of the antibody value in blood had been confirmed by solid phase enzyme immunoassay using 96 wellplates, PBS having, dissolved therein Cu,Zn-SOD derived from human erythrocytes was injected intravenously into the tail of the mouse. After 4 days, spleen cells of the mouse were collected, and these cells were fused with 8-azaguanine-resistant myeloma cells SP2/0-Ag14 by using polyethylene glycol. These cells were cultured on 96 wellplates, and the known HAT selection was carried out. The hybridoma was screened by solid phase enzyme immunoassay using 96 wellplates. With respect to a hypridoma producing an antibody to human SOD, cloning was carried out according to the limiting dilution method. The thus-obtained hybridoma was cultured in a flask and transplanted into the abdominal cavity of BALB/c mouse, followed by culturing, to obtain ascitic fluid containing a monoclonal antibody. The abdominal dropsy was subjected to ammonium sulfate precipitation and ion exchange column chromatography to obtain a monoclonal antibody to human Cu,Zn-SOD. This monoclonal antibody was specific to human Cu,Zn-SOD, and it was confirmed that the monoclonal antibody had no reactivity with Zn-SOD and Mn-SOD of other animals such as bovine, swine and rat.

(B) Preparation of Immobilized Monoclonal Antibody

To each well of a 96-well microtiter plate (immunoplate supplied by MUNC) was added 250 $\mu$l of 0.1M sodium carbonate buffer (pH = 9.6) containing 1 $\mu$g/ml of the mouse monoclonal antibody to human Cu,Zn-SOD [the monoclonal antibody prepared in (A) above; hereinafter referred to as "SH2B"; IgGl], and incubation was carried out at 4°C overnight. The solution was removed from each cell, and each cell was washed three times with PBS solution containing 0.04% of Tween 20 (hereinafter referred to as "PBS-T solution") and 300 $\mu$l of PBS-T solution containing 1% of BSA (bovine serum albumin) was added to each well. The blocking treatment (the treatment for making BSA adsorbed in non-specific bonding sites to the antigen or antibody, present on the carrier) was carried out at 4°C, and the monoclonal antibody-immobilized microtiter plate was stored in this state at 4°C.

(C) Preparation of Labelled Monoclonal Antibody

To 1 ml of a solution of 5 mg/ml of horseradish peroxidase (hereinafter referred to as "HRP") in 0.3M sodium bicarbonate buffer (pH = 8.1) was added 0.1 ml of a 1% solution of 1-fluoro-2,4-dinitrobenzene in ethanol, and reaction was carried out at room temperature for 1 hour. Then, 1.0 ml of 0.06M sodium periodate was added to the reaction mixture and was reacted for 30 minutes. Unreacted sodium periodate was removed by addition of 1.0 ml of 0.16M ethylene glycol, and the reaction mixture was dialyzed against 0.01M sodium carbonate buffer (pH = 9.5).

Then, 5 mg of the same monoclonal antibody (SH2B) as used for the immobilized antibody was added to the dialyzate and reaction was carried out for 5 hours. Then, 5 mg of sodium borohydride was added to the reaction mixture, and the mixture was allowed to stand at 4°C overnight.

The thus-obtained reaction product was purified by the high-performance liquid chromatography using TSK Gel G-3000SW (trademark for the product supplied by TOSOH Corp.), whereby a monoclonal antibody labelled with HRP was obtained.

(D) Measurement of Human Cu,Zn-SOD by Enzyme Immunoassay

The antibody-immobilized microtiter plate prepared in (B) of the present example was returned to room temperature, and the microtiter plate was washed three times with the PBS-T solution. Then, 20 $\mu$l of normal rabbit serum containing 0 to 320 ng/ml of Cu,Zn-SOD derived from purified human erythrocytes [SDS-electrophoretically homogeneous reference product prepared from human erythrocytes and having an activity of 3,500 units per mg of the protein concentration determined by the Lawry method using bovine serum albumin as the reference substance (1 unit determined by the method of Mc'Cord; J. Biol. Chem., 244, page 6049, 1969)] was added as the reference substance to each well. Then, 300 $\mu$l of a solution obtained by diluting 1000 times 4.5 mg/ml of the HRP-labelled antibody prepared in (C) above with the PBS-T solution containing 1.5% of normal rabbit serum, 0.01% of myoglobin, 0.075% of refined white sugar and 0.05% of egg albumin was added to each well, and incubation was carried out at room temperature for 3 hours. The solution was removed and each well was washed three times with the PBS-T solution. Then, 200 $\mu$l of 0.1M citrate buffer (pH = 4.1) containing 0.03% of ABTS and 0.01% of $H_2O_2$ was added to each well, and reaction was carried out at room temperature for 30 minutes. The reaction was stopped by addition of 100 $\mu$l of 1M oxalic acid to each well. After the reaction was stopped, with respect to each well, the absorption intensity at a wavelength of 415 nm and the absorption intensity at a reference wavelength of 492 nm were measured by an automatic microtiter plate reader (MPR-A4 supplied by TOSOH Corp.). The concentration of the reference substance and the adsorption intensity were plotted to obtain the results shown in Table 1, and a calibration curve was prepared. By using this calibration curve, the human Cu,Zn-SOD concentration in each sample could be obtained.

Table 1

| Concentration of Human Cu,Zn-SOD in Serum (ng/ml) | Absorbance at 415 nm (reference wavelength = 492 nm) |
|---|---|
| 0.0 | <0.01 |
| 1.00 | 0.05 |
| 2.5 | 0.09 |
| 5.0 | 0.21 |
| 10.0 | 0.43 |
| 20.0 | 0.81 |
| 40.0 | 1.28 |
| 80.0 | 2.01 |
| 160.0 | 2.34 |
| 320.0 | 2.61 |

(E) Examination of Cross Reactivity of SH2B

The presence or absence of the cross reactivity of the mouse monoclonal antibody SH2B to human Cu,Zn-SOD was examined by repeating the procedures of (D) of the present example using normal rabbit serum containing 320 ng/ml of Cu,Zn-SOD derived from bovine, swine or rat erythrocytes or Mn-SOD derived from mitochondria instead of the human Cu,Zn-SOD which was used as the reference substance. In each case, the absorbance at 415 nm was less than 0.01. Accordingly, it was confirmed that the reagent for the solid phase immunoassay according to the present example, that is, the combination of the immobilized antibody and labelled antibody, was specific to human Cu,Zn-SOD.

Example 2

(A) Preparation of Monoclonal Antibody to Bovine Cu,Zn-SOD

A mouse monoclonal antibody to bovine Cu,Zn-SOD was prepared by repeating the procedures of (A) of Example 1 in the same manner except that Cu,Zn-SOD derived from bovine erythrocytes instead of the human Cu,Zn-SOD was used as the antigen. It was confirmed that the monoclonal antibody was specific to bovine Cu,Zn-SOD and did not show any reactivity with Cu,Zn-SOD and Mn-SOD of other animals, for example, human, swine and rat.

(B) Preparation of Immobilized Monoclonal Antibody

An immobilized monoclonal antibody was prepared in the same manner as described in (B) of Example 1 using the mouse monoclonal antibody to bovine Cu,Zn-SOD prepared in (A) of the present example (hereinafter referred to as "SH3K"; IgGl)

(C) Preparation of Labelled Monoclonal Antibody

A labelled monoclonal antibody was prepared in the same manner as described in (C) of Example 1 using the same monoclonal antibody as prepared in (A) of the present example and used for the immobilized monoclonal antibody prepared in (B) of the present example.

(D) Determination of Bovine Cu,Zn-SOD by Enzyme Immunoassay

The procedures of (D) of Example 1 were repeated in the same manner by using the immobilized monoclonal antibody and labelled monoclonal antibody prepared in (B) and (C), respectively, of the present example and normal rabbit serum containing 0 to 320 ng/ml of bovine Cu,Zn-SOD as the reference substance. The bovine Cu,Zn-SOD used was a reference product derived from bovine erythrocytes and refined so that it was regarded as being homogeneous at the SDS electrophoresis. This reference substance had an activity of 3,800 units per mg of the protein concentration determined by the Lawry method using bovine serum albumin as the reference substance (1 unit determined by the method of Mc'Cord; J. Biol. Chem., 244, page 6049, 1969). The concentration of bovine Cu,Zn-SOD and the absorption intensity were plotted to obtain results shown in Table 2, and a calibration curve was prepared. The bovine Cu,Zn-SOD concentration in each sample could be determined by using this calibration curve.

Table 2

| Concentration of Bovine Cu,Zn-SOD in Serum (ng/ml) | Absorbance at 415 nm (reference wavelength = 492 nm) |
|---|---|
| 0.0 | <0.01 |
| 1.00 | 0.02 |
| 2.5 | 0.05 |
| 5.0 | 0.08 |
| 10.0 | 0.12 |
| 20.0 | 0.29 |
| 40.0 | 0.55 |
| 80.0 | 1.25 |
| 160.0 | 1.88 |
| 320.0 | 2.50 |

(E) Examination of Cross Reactivity of SH3K

The presence or absence of the cross reactivity of the monoclonal antibody SH3K was examined by repeating the procedures of (D) of the present example using normal rabbit serum containing 320 ng/ml of Cu,Zn-SOD derived from human, swine or rat erythrocytes or Mn-SOD derived from mitochondria instead of the bovine Cu,Zn-SOD which was used as the reference substance. In each case, the absorbance at 415 nm was less than 0.01. Accordingly, it was confirmed that the kit of the present example was specific to bovine Cu,Zn-SOD.

From the foregoing results, it will be understood that the method of the present invention is excellent and valuable as the method for selectively detecting and measuring Cu,Zn-SOD in various samples.

**Claims**

1. A method of measuring Cu,Zn-superoxide dismutase, which comprises placing in a vessel a sample containing Cu,Zn-superoxide dismutase at a concentration of from 1 ng/ml to 320 ng/ml and performing in the vessel the antigen-antibody reaction of a monoclonal antibody specifically recognizing Cu,Zn-superoxide dismutase, which is immobilized on a solid carrier, and, simultaneously therewith, an antigen-antibody reaction of the same Cu,Zn-superoxide dismutase-recognizing monoclonal antibody, which is labelled with a marker.

2. The method according to claim 1, wherein the monoclonal antibodies specifically recognizing Cu,Zn-superoxide dismutase are prepared by fusing antibody-producing spleen cells of an animal immunized with Cu,Zn-superoxide dismutase to myeloma cells to obtain hybridoma producing monoclonal antibodies recognizing Cu,Zn-superoxide dismutase, culturing the hybridoma or cell strains derived therefrom, and collecting the thus-produced monoclonal antibodies from the cultured products.

3. A monoclonal antibody specifically recognizing Cu,Zn-superoxide dismutase.